# EUROPEAN PATENT SPECIFICATION

(11) **EP 2 392 307 B1**
(45) Date of publication and mention of the grant of the patent: **04.03.2015**
(21) Application number: 10736068.7
(22) Date of filing: 02.02.2010
(51) Int. Cl.: A61J 3/00, B65D 83/00, A61M 5/168, A61M 39/22, F16K 31/04, A61M 5/14, F16K 11/085

(54) **DRUG SOLUTION DOSE CONTROLLER**
STEUERGERÄT ZUR ARZNEIMITTELLÖSUNGSDOSIERUNG
DISPOSITIF DE COMMANDE DE DOSE DE SOLUTION DE MÉDICAMENT

(30) Priority: 02.02.2009 KR 20090008090
(43) Date of publication of application: 07.12.2011
(73) Proprietor: Woo Young Medical Co., Ltd., Jincheon-gun, Chungcheongbuk-do 365-801 (KR)
(72) Inventor: LEE, Young Gyu, Seoul 135-841 (KR)
(74) Representative: Maser, Jochen
(86) International application number: PCT/KR2010/000616
(87) International publication number: WO 2010/087677

(56) References cited:
- JP-A- 5 084 310
- JP-A- 9 280 394
- JP-A- 2007 236 964
- US-A- 4 620 690
- US-A1- 2005 267 418
- US-A1- 2008 177 233

## Description

### Technical Field

The present invention relates to an apparatus for controlling an administration rate (flow rate) of medical fluid, such as an anodyne, an antibiotic, etc., when it is administered to an administration target (for example, a patient).

### Background Art

Generally, special antibiotics for cancer treatments are administered to cancer patients. In the case of patients who require pain management, anodynes are administered to them to reduce pain. Unlike general medicines, administering such special antibiotics or anodynes may lead to a coma or shock death of a patient through overdosing on medical fluid. On the other hand, if the administration amount of such medical fluid is small, it is difficult to achieve the intended purpose of the medical fluid. Therefore, it is very important to appropriately control the administration amount of medical fluid within a permissible range.

Hitherto, syringes have been typically used to administer medical fluid to administration targets. However, because the administration rate of medical fluid is determined depending on conditions such as force with which a user presses a syringe, mastery of the user, etc., it is very difficult to maintain the administration rate of medical fluid to be constant. As another way to administer medical fluid to an administration target, a flow rate control device is provided on a hose for administration of medical fluid so as to enable a user to control the administration rate of medical fluid while checking the administration rate with the naked eye. However, this way is disadvantageous in that it is difficult to administer an appropriate required amount of medical fluid to a patient in response to conditions of the patient which vary frequently.

An apparatus for controlling an administration rate of medical fluid is disclosed by US 2005/0267418 A1. This apparatus comprises a valve housing having a cylindrical valve chamber with a plurality of fluid inlet and a fluid outlet. Further, there is provided a conversion valve rotatably contained in the valve chamber having a passage communicating at least one of the medical fluid inlets with the outlets depending on an angle at which the conversion valve rotates. This conversion valve is rotated by a valve control unit.

US 2008/017723 A1 discloses an apparatus for controlling an administration rate of a medical fluid having a valve housing with a cylindrical valve chamber with a plurality of medical fluid inlets and a medical fluid outlet. Further, there is provided a medical fluid branch unit dividing medical fluid supplied from a fluid source into a plurality of streams of medical fluid into the respective fluid inlet of the valve housing.

A modular flow control cassette is disclosed by US 4,620,690. This modular flow control cassette comprises a house carrying a flow tube which passes between a pair of arms. The flow tube is pinched by turning an adjusting nut rotating the pair of arms. This adjusting nut can be driven by a control module.

### Disclosure

### Technical Problem

Accordingly, the present invention has been made keeping in mind the above problems occurring in the prior art, and an object of the present invention is to provide an apparatus for precisely controlling an administration rate of medical fluid.

### Technical Solution

In order to accomplish the above object, the present invention provides an apparatus for controlling an administration rate of medical fluid, including: a valve housing having a cylindrical valve chamber therein, with a plurality of medical fluid inlets and a medical fluid outlet formed in a circumferential surface of the valve chamber; a medical fluid branch unit dividing medical fluid supplied from a medical fluid source into a plurality of streams of medical fluid having different flow rates and supplying the plurality of streams of medical fluid into the respective medical fluid inlets; a conversion valve rotatably contained in the valve chamber, the conversion valve having a passage communicating at least one of the medical fluid inlets with the medical fluid outlet depending on an angle at which the conversion valve rotates; and a valve control unit rotating the conversion valve.

The medical fluid branch unit includes: an inlet tube into which medical fluid is supplied from the medical fluid source; and a plurality of flow rate control tubes diverging from the inlet tube, the flow rate control tubes being connected to the respective medical fluid inlets, with passages defined in the respective flow rate control tubes, the passages having different cross-sectional areas. Preferably, each of the passages of the flow rate control tubes may comprise a capillary passage.

The medical fluid inlets and the medical fluid outlet may be disposed opposite to each other. The passage of the conversion valve may comprise a first passage through which the streams of medical fluid supplied from the medical fluid inlets flow into the medical fluid outlet. When the conversion valve is rotated 180°, the first passage may be converted to a second passage which prevents the streams of medical fluid supplied from the medical fluid inlets from flowing into the medical fluid outlet.

The passage of the conversion valve may further comprise a partially open passage through which medical fluid supplied from some of the medical fluid inlets flows into the medical fluid outlet.

The medical fluid inlets may comprise first and second inlets. The passage of the conversion valve may comprise a third passage through which medical fluid supplied from the first inlet flows into the medical fluid outlet, and a fourth passage which prevents medical fluid supplied from the second inlet from flowing into the outlet while the medical fluid flows from the first inlet into the medical fluid outlet through the third passage.

The medical fluid inlets and the medical fluid outlet may be disposed opposite to each other. When the conversion valve is rotated 180°, the third passage and the fourth passage may be respectively converted to a fifth passage preventing medical fluid supplied from the first inlet from flowing into the outlet, and a sixth passage through which medical fluid supplied from the second inlet flows into the outlet.

### Advantageous Effects

The present invention can control the administration rate of a medical fluid so that it is administered to an administration target at an appropriate administration rate.

### Description of Drawings

Figs. 1 and 2 are, respectively, a perspective view and an exploded perspective view showing an apparatus for controlling an administration rate of medical fluid, according to a first embodiment of the present invention;
Fig. 3 is a sectional view taken along line A-A of Fig. 1;
Fig. 4 is an exploded sectional view of a valve housing of Fig. 3;
Figs. 5 and 6 are, respectively, sectional perspective views taken along lines B-B and C-C of Fig. 2;
Figs. 7 through 10 are sectional views showing the operation of the administration rate control apparatus according to the first embodiment;
Figs. 11 and 12 are, respectively, an exploded perspective view and a front view showing an example of application of the administration rate control apparatus according to the first embodiment;
Fig. 13 is an exploded perspective view showing an apparatus for controlling an administration rate of medical fluid, according to a second embodiment of the present invention.

### Best Mode

Hereinafter, preferred embodiments of the present invention will be described in detail with reference to the attached drawings. For reference, in the drawings, the thicknesses of lines or the sizes of elements may be exaggerated to more clearly and conveniently illustrate the present invention. Furthermore, the terms used will be defined in consideration of their functions in the present invention, and thus the definition of these terms may be changed depending on the intention or practice of a user or operator. Therefore, the definitions of the terms should be determined based on the whole content of the specification.

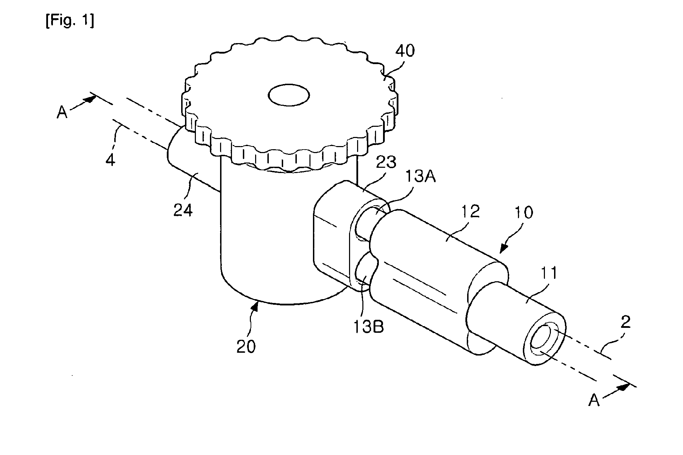

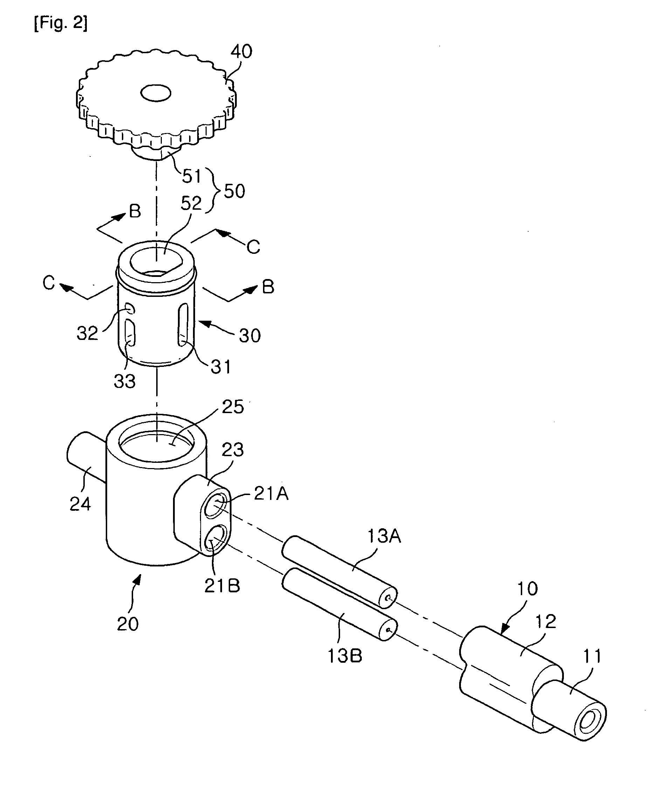

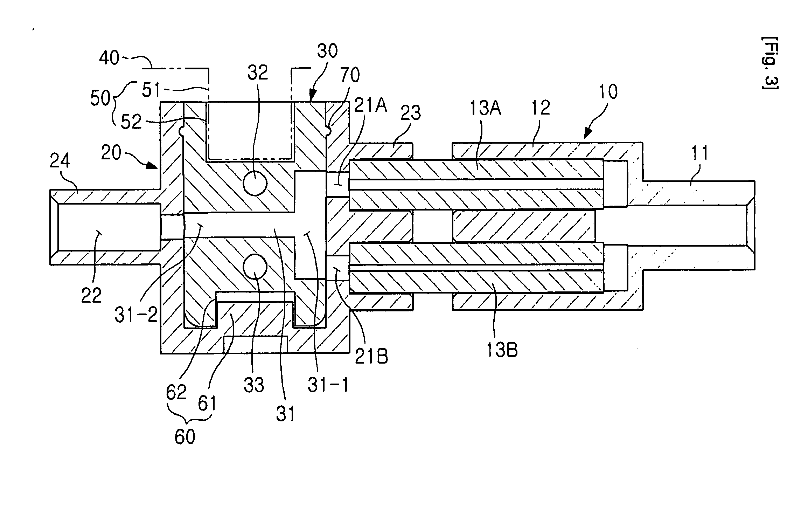

Figs. 1 and 2 are, respectively, a perspective view and an exploded perspective view showing an apparatus for controlling an administration rate of medical fluid, according to a first embodiment of the present invention. Fig. 3 is a sectional view taken along line A-A of Fig. 1. As shown in Figs. 1 through 3, the administration rate control apparatus according to the first embodiment of the present invention includes a medical fluid branch unit 10, a valve housing 20, a conversion valve 30, and a control handle 40. The medical fluid branch unit 10 receives medical fluid from a medical fluid supply source and divides the medical fluid into a plurality of streams. The valve housing 20 has a plurality of inlets 21A and 21B into which the streams of medical fluid are respectively supplied from the medical fluid branch unit 10, and an outlet 22 through which the medical fluid is discharged from the valve housing 20. The conversion valve 30 has a plurality of medical fluid passages 31, 32 and 33, and is installed in the valve housing 20. The conversion valve 30 selectively communicates both or one of the inlets 21A and 21B with the outlet 22 through one of the medical fluid passages 31, 32 and 33 or interrupts the communication therebetween. The control handle 40 is a valve control unit which operates the conversion valve 30.

The administration rate control apparatus according to the first embodiment is provided on a line (see reference numerals 2 and 4) which extends from the medical fluid source to an injection unit (such as an injection needle, a catheter, etc.) which is directly connected to an administration target (for example, a patient). Thus, when at least one of the inlets 21A and 21B communicates with the outlet 22 through one of the medical fluid passages 31, 32 and 33, medical fluid can be administrated from the medical fluid source to the administration target. When the communication between at least one of the inlets 21A and 21B and the outlet 22 is interrupted, the administration of medical fluid stops.

The medical fluid branch unit 10 includes an inlet tube 11 into which medical fluid is supplied from the medical fluid source, and two flow rate control tubes 13A and 13B which divides the medical fluid that has been supplied from the inlet tube 11 into two streams having different flow rates.

Furthermore, an inlet hose 2 is connected to one end of the inlet tube 11. The inlet hose 2 functions as an inlet line which transmits from the medical fluid source to the medical fluid branch unit 10. A connector 12 is provided on the other end of the inlet tube 11. The connector 12 has two coupling holes. The two coupling holes are parallel to each other and form a bifurcate structure in which a passage of the inlet tube 11 bifurcates into the two coupling holes. Thus, the inlet tube 11 and the connector 12 form a Y-shaped connector structure.

The flow rate control tubes 13A and 13B are respectively inserted into the two coupling holes. Medical fluid supplied from the inlet tube 11 is drawn into the flow rate control tubes 13A and 13B. Preferably, the flow rate control tubes 13A and 13B are coupled to the connector 12 such that medical fluid is prevented from leaking out. As necessary, the connector 12 may be integrated with the flow rate control tubes 13A and 13B.

Each of the two flow rate control tubes 13A and 13B has a capillary passage. Furthermore, the passages of the two flow rate control tubes 13A and 13B have different cross-sectional areas (different diameters) so that the fluxes (flow rates) of the streams of medical fluid which flow along the two flow rate control tubes 13A and 13B are different. In this embodiment, the passage of the flow rate control tube of reference numeral 13A is larger than the passage of the flow rate control tube of reference numeral 13B (refer to Fig. 3).

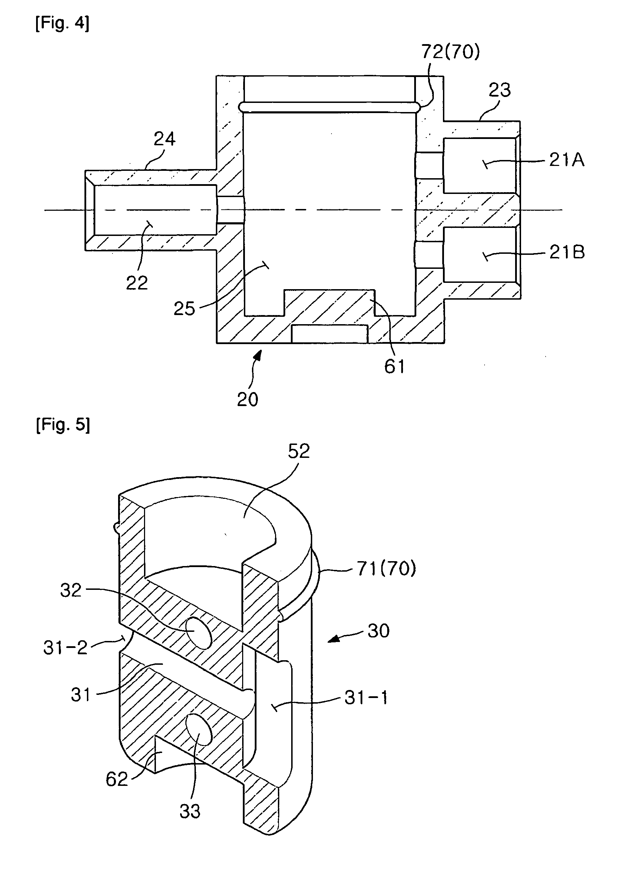

Fig. 4 is an exploded sectional view of the valve housing 20 of Fig. 3. The valve housing 20 has a valve chamber 25 which has a vertically cylindrical shape and contains the conversion valve 30 therein. The valve chamber 25 is open on a top end thereof so that the conversion valve 30 contained in the valve chamber 25 is exposed to the outside through the open top end of the valve chamber 25. An inlet connection port 23 and an outlet connection port 24 which communicate with the valve chamber 25 are provided on a circumferential outer surface of the valve housing 20.

The inlet connection port 23 has two passages which are parallel to each other and are spaced apart from each other in a vertical direction by a predetermined distance. The two passages of the inlet connection port 23 respectively function as the inlets 21A and 21B. Thus, the valve housing 20 has the two inlets 21A and 21B which are spaced apart from each other in a vertical direction.

The flow rate control tubes 13A and 13B are respectively inserted into the passages of the inlet connection port 23. Because the two passages of the inlet connection port 23 are disposed at upper and lower positions spaced apart from each other in a vertical direction, the two flow rate control tubes 13A and 13B coupled to the inlet connection port 23 are disposed in the same manner as that of the two passages of the inlet connection port 23. Thus, medical fluid which flows through the flow rate control tube 13A disposed at the upper position is drawn into the upper inlet 21A, and medical fluid which flows through the flow rate control tube 13B disposed at the lower position is drawn into the lower inlet 21B. Preferably, the flow rate control tubes 13A and 13B are also coupled to the inlet connection port 23 such that medical fluid is prevented from leaking out of them. As necessary, the flow rate control tubes 13A and 13B may also be integrated with the inlet connection port 23.

The outlet connection port 24 is disposed opposite the inlet connection port 23 with respect to the valve chamber 25. A passage of the outlet connection port 24 functions as the outlet 22. Thus, the outlet 22 is opposite the inlets 21A and 21B. In addition, an outlet hose 4 which functions as the outlet line is connected to the outlet connection port 24 to provide medical fluid from the outlet connection port 24 to the administration target. Compared to the two inlets 21A and 21B disposed at upper and lower positions, the outlet 22 is disposed at a position lower than the upper inlet 21A but higher than the lower inlet 21B. In other words, the outlet 22 is disposed at a height between the two inlets 21A and 21B.

The conversion valve 30 has a cylindrical shape exactly corresponding to that of the valve chamber 25 and is disposed in the valve chamber 25 so as to be rotatable around the center axis of the valve chamber 25. The control handle 40 is removably coupled to an exposed top end of the conversion valve 30 so that when the control handle 40 rotates to the left or right, the conversion valve 30 rotates in the same direction.

Here, a coupling rod 51 is provided on either the conversion valve 30 or the control handle 40, and a coupling depression 52 into which the coupling rod 51 is fitted is formed in the other of the conversion valve 30 and the control handle 40. The coupling rod 51 and the coupling depression 52 constitute a removable coupling means 50. In this embodiment, the coupling rod 51 is provided under a bottom end of the control handle 40, and the coupling depression 52 is formed in the top end of the conversion valve 30.

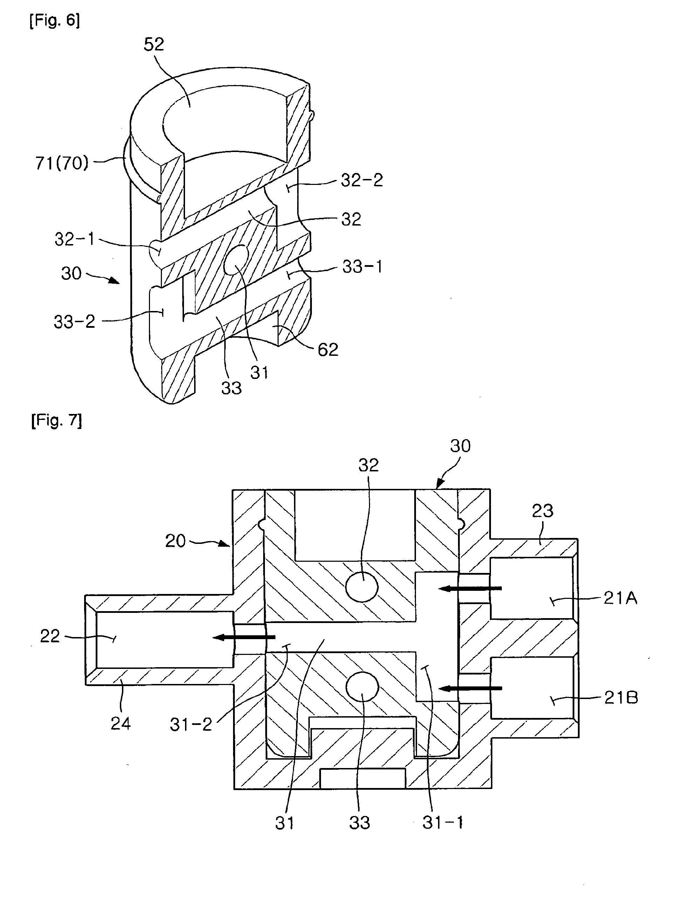

Figs. 5 and 6 are, respectively, sectional perspective views taken along line B-B and C-C of Fig. 2 to show the medical fluid passages 31, 32 and 33 of the conversion valve 30. As shown in Figs. 5 and 6, the three medical fluid passages 31, 32 and 33 are provided in this embodiment. Each medical fluid passage 31, 32, 33 is configured in such a way that the cross-sectional area thereof is constant in the overall length other than at one end. In addition, other than at these ends, the cross-sectional areas of the medical fluid passages 31, 32 and 33 are the same. The three medical fluid passages 31, 32 and 33 are formed through the conversion valve 30 in directions perpendicular to the rotational center axis of the conversion valve 30. Both open ends of each of the medical fluid passages 31, 32 and 33 are respectively oriented towards the inlets 21A and 21B and the outlet 22 depending on an angle at which the conversion valve 30 rotates. Hereinafter, the three medical fluid passages 31, 32 and 33 will be respectively designated as first, second and third medical fluid passages, and both ends of each of the medical fluid passages 31, 32 and 33 will be respectively designated as first and second ports.

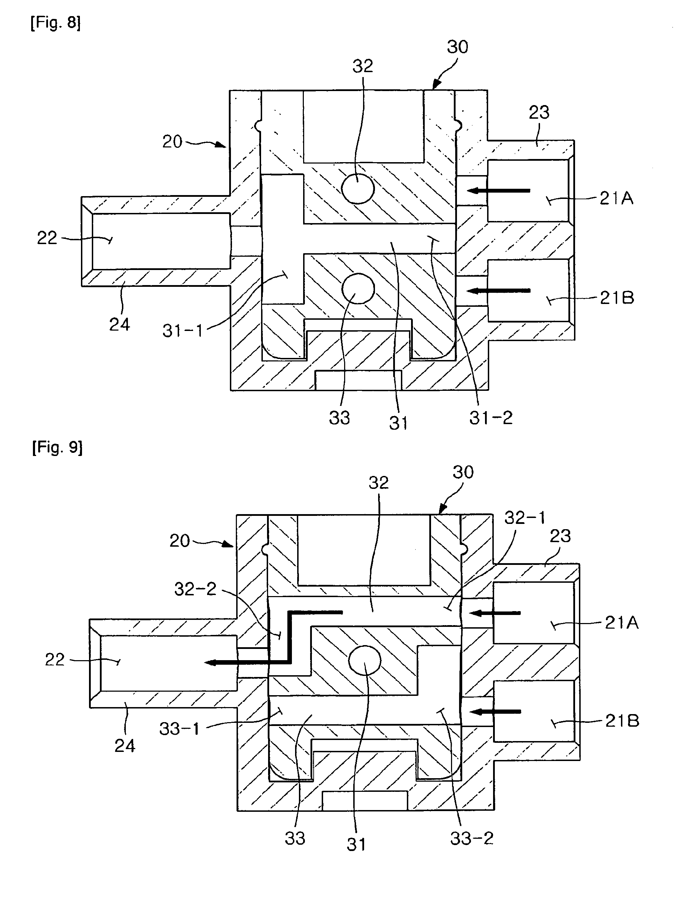

When the first medical fluid passage 31 is oriented such that a first port 31-1 thereof faces the two inlets 21A and 21B and a second port 31-2 thereof faces the outlet 22, the first port 31-1 communicates with the inlets 21A and 21B and simultaneously the second port 31-2 communicates with the outlet 22 (refer to Fig. 7). On the other hand, when the conversion valve 30 is rotated 180° and is thus oriented such that the first port 31-1 faces the outlet 22 and the second port 31-2 faces the two inlets 21A and 21B, the second port 31-2 communicates with neither the upper inlet 21A nor the lower inlet 21B (refer to Fig. 8).

In detail, the first medical fluid passage 31 is aligned with the outlet 22 so that when the second port 31-2 of the first medical fluid passage 31 is oriented towards the outlet 22, the first medical fluid passage 31 communicates with the outlet 22. The cross-sectional area of the first medical fluid passage 31 is constant in the overall length other than at the first port 31-1 thereof, and the first medical fluid passage 31 has a size that is appropriate for being disposed between the two inlets 21A and 21B such that the second port 31-2 does not even partially communicate with the upper inlet 21A or the lower inlet 21B when the second port 31-2 is oriented towards the inlets 21A and 21B. The first port 31-1 of the first medical fluid passage 31 has a vertically extended structure so that it can communicate with the two inlets 21A and 21B together.

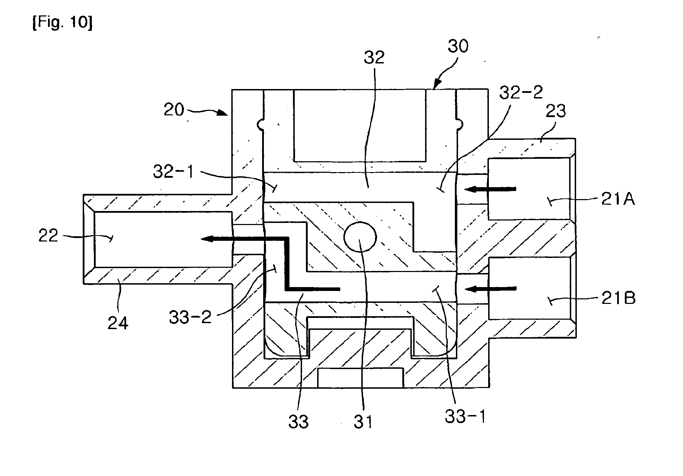

The second medical fluid passage 32 passes through the conversion valve 30 in a direction perpendicular to the first medical fluid passage 31. Thus, when the conversion valve 30 rotates 90° from the state in which the first and second ports 31-1 and 31-2 of the first medical fluid passage 31 are oriented towards the inlets 21A and 21B and the outlet 22, first and second ports 32-1 and 32-2 of the second medical fluid passage 32 are oriented towards the inlets 21A and 21B and the outlet 22. In the case of the second medical fluid passage 32, when the first port 32-1 is oriented towards the inlets 21A and 21B and the second port 32-2 is oriented towards the outlet 22, the first port 32-1 communicates with the upper inlet 21A and, simultaneously, the second port 32-2 communicates with the outlet 22 (refer to Fig. 9). On the other hand, when the conversion valve 30 rotates 180° from the above state so that the first port 32-1 is oriented towards the outlet 22 and the second port 32-2 is oriented towards the two inlets 21A and 21B, the first port 32-1 cannot communicate with the outlet 22 (refer to Fig. 10).

In more detail, the second medical fluid passage 32 is aligned with the upper inlet 21A so that when the first port 32-1 of the second medical fluid passage 32 is oriented towards the two inlets 21A and 21B, it communicates with the upper inlet 21A, but when the first port 32-1 of the second medical fluid passage 32 is oriented towards the outlet 22, it cannot communicate with the outlet 22 because the outlet 22 is disposed at a lower position than the inlet 21A. The cross-sectional area of the second medical fluid passage 32 is constant in the overall length other than at the second port 32-2. The second port 32-2 has a shape which extends downwards from the second medical fluid passage 32 so that it can communicate with the outlet 22.

The third medical fluid passage 33 has first and second ports 33-1 and 33-2 and passes through the conversion valve 30 in a direction parallel to the second medical fluid passage 32. Thus, when the conversion valve 30 is oriented such that the first and second ports 32-1 and 32-2 of the second medical fluid passage 32 are oriented towards the inlets 21A and 21B and the outlet 22, the first and second ports 33-1 and 33-2 of the third medical fluid passage 33 are also oriented towards the inlets 21A and 21B and the outlet 22 in the same way as those of the second medical fluid passage 32.

In the case of the third medical fluid passage 33, when the first port 32-1 of the second medical fluid passage 32 is oriented towards the outlet 22 and the second port 32-2 of the second medical fluid passage 32 is oriented towards the two inlets 21A and 21B, the first port 33-1 of the third medical fluid passage 33 is oriented towards the two inlets 21A and 21B and, simultaneously, the second port 33-2 thereof is oriented towards the outlet 22. On the contrary to this, when the conversion valve 30 rotates 180° from the above state so that the first port 32-1 of the second medical fluid passage 32 is oriented towards the two inlets 21A and 21B and the second port 32-2 of the second medical fluid passage 32 is oriented towards the outlet 22, the first port 33-1 of the third medical fluid passage 33 is oriented towards the outlet 22 and, simultaneously, the second port 33-2 thereof is oriented towards the two inlets 21A and 21B.

Furthermore, when the first port 33-1 of the third medical fluid passage 33 is oriented towards the two inlets 21A and 21B and the second port 33-2 thereof is oriented towards the outlet 22, the first port 33-1 communicates with the lower inlet 21B and, simultaneously, the second port 33-2 communicates with the outlet 22 (refer to Fig. 10). On the other hand, when the first port 33-1 of the third medical fluid passage 33 is oriented towards the outlet 22 and the second port 33-2 thereof is oriented towards the two inlets 21a and 21B, the first port 33-1 cannot communicate with the outlet 22 (refer to Fig. 9).

In more detail, the third medical fluid passage 33 is aligned with the lower inlet 21B so that when the first port 33-1 of the third medical fluid passage 33 is oriented towards the two inlets 21A and 21B, the first port 33-1 communicates with the lower inlet 21B, but when the first port 33-1 of the third medical fluid passage 33 is oriented towards the outlet 22, the first port 33-1 cannot communicate with the outlet 22 because the outlet 22 is disposed at a higher position than the lower inlet 21B. The cross-sectional area of the third medical fluid passage 33 is constant in the overall length other than at the second port 33-2. The second port 32-2 has a shape which extends upwards from the third medical fluid passage 33 so that it can communicate with the outlet 22.

In the case of the above-stated conversion valve 30 having the first through third medical fluid passages 31, 32, and 33, a user can select one of four modes to control the administration rate of medical fluid administered to the administration target.

One of the four modes is an entirely open mode in which both streams of medical fluid which are supplied from the flow rate control tubes 13A and 13B via the two inlets 21A and 21B are administered to the administration target. Another mode is an entirely closed mode in which both streams of medical fluid which are supplied from the flow rate control tubes 13A and 13B are interrupted so that the administration of medical fluid stops. These two modes are embodied by the first medical fluid passage 31.

Another mode is a first partially open mode in which only medical fluid that is supplied from the upper flow rate control tube 13A via the upper inlet 21A is administered to the administration target. The other mode is a second partially open mode in which only medical fluid that is supplied from the lower flow rate control tube 13B via the lower inlet 21B is administered to the administration target. These two partially open modes are embodied by the second and third medical fluid passages 32 and 33. Moreover, the administration rate of medical fluid when in the first partially open mode is greater than that of the second partially open mode because the passage of the upper flow rate control tube 13A is wider than that of the lower flow rate control tube 13B.

For reference, in this embodiment, from the entirely open mode, each time the conversion valve 30 is rotated to the right at an interval of 90° by manipulating the control handle 40, the administration mode is converted in a sequence of the second partially open mode (in which medical fluid flows only through the lower flow rate control tube 13B), the entirely closed mode, and the first partially open mode.

The operation of the first embodiment will be described with reference to Figs. 7 through 10.

Fig. 7 illustrates the entirely open mode. As shown in Fig. 7, in the entirely open mode, the first port 31-1 of the first medical fluid passage 31 communicates with the two inlets 21A and 21B, and the second port 31-2 of the first medical fluid passage 31 communicates with the outlet 22. Two streams of medical fluid which are supplied from the two flow rate control tubes 13A and 13B are drawn into the two inlets 21A and 21B and then flow through the first medical fluid passage 31 before being discharged through the outlet 22. In this mode, the first medical fluid passage 31 forms a first passage through which the sum of the two streams of medical fluid supplied from the flow rate control tubes 13A and 13B is administered to the administration target.

In this state, when the conversion valve 30 is rotated 180° by manipulating the control handle 40, the administration mode is converted from the entirely open mode to the entirely closed mode. Fig. 8 illustrates the entirely closed mode. As shown in Fig. 8, in the entirely closed mode, the second port 31-2 of the first medical fluid passage 31 communicates with neither the upper inlet 21A nor the lower inlet 21B so that the two streams of medical fluid supplied from the two flow rate control tubes 13A and 13B cannot enter the upper inlet 21A or the lower 21B. In this mode, the first medical fluid passage 31 forms a second passage which interrupts the administration of medical fluid.

When the conversion valve 30 is rotated to the left 90° from the entirely open mode or when the conversion valve 30 is rotated to the right 90° from the entirely closed mode, the administration mode is converted to the first partially open mode. Fig. 9 illustrates the first partially open mode. As shown in Fig. 9, in the first partially open mode, the first port 32-1 of the second medical fluid passage 32 communicates with the upper inlet 21A and the second port 32-2 of the second medical fluid passage 32 communicates with the outlet 22. In this mode, medical fluid that is supplied from the flow rate control tube 13A successively flows through the upper inlet 21A and the second medical fluid passage 32 before being discharged through the outlet 22. On the other hand, the first port 33-1 of the third medical fluid passage 33 cannot communicate with the outlet 22 so that medical fluid that is supplied from the lower flow rate control tube 13B is prevented from being discharged through the outlet 22. In this mode, the second medical fluid passage 32 forms a third passage through which medical fluid that flows along the upper flow rate control tube 13A is administered to the administration target. The third medical fluid passage 33 forms a fourth passage which interrupts administration of medical fluid that flows through the lower flow rate control tube 13B.

Meanwhile, when the conversion valve 30 is rotated to the right 90° from the entirely open mode or when the conversion valve 30 is rotated to the left 90° from the entirely closed mode, or when the conversion valve 30 is rotated 180° from the first partially open mode, the administration mode is converted to the second partially open mode. Fig. 10 illustrates the second partially open mode. As shown in Fig. 10, in the second partially open mode, the first port 33-1 of the third medical fluid passage 33 communicates with the lower inlet 21B and the second port 33-2 of the third medical fluid passage 33 communicates with the outlet 22. Thus, medical fluid that is supplied from the lower flow rate control tube 13B successively flows through the lower inlet 21B and the third medical fluid passage 33 before being discharged through the outlet 22. On the other hand, the first port 32-1 of the second medical fluid passage 32 cannot communicate with the outlet 22 so that medical fluid that is supplied from the upper flow rate control tube 13A is prevented from being discharged through the outlet 22. In this mode, the second medical fluid passage 32 forms a fifth passage which interrupts administration of medical fluid that flows through the upper flow rate control tube 13A, and the third medical fluid passage 33 forms a sixth passage through which medical fluid that flows along the lower flow rate control tube 13B is administered to the administration target.

The administration rate control apparatus of the first embodiment that is operated in the above-mentioned manner is very useful, as will be explained.

In the case of surgical patients, although there is a difference in degree of pain depending on the kind of surgical operation or a difference between individuals, a patient generally feels severe pain at an initial stage after a surgical operation, over time the pain is gradually relieved, and after three days, the pain is greatly reduced. Out of consideration of this, when medical fluid for pain management is administered to the surgical patient, the administration rate of medical fluid can be reduced in stages. In detail, in the initial stage after the surgical operation, the entirely open mode (in which the sum of the streams of medical fluid that flow through both of the two flow rate control tubes 13A and 13B is administered to the patient) is selected. After the pain is somewhat reduced, the administration mode is converted to the first partially open mode (in which only medical fluid that flows through the upper flow rate control tube 13A is administered to the patient). After three days, the administration mode is converted to the second partially open mode (in which only medical fluid that flows through the lower flow rate control tube 13B is administered to the patient).

In the case of a patient who has terminal cancer, as time passes, the pain increases, contrary to the surgical patient. Hence, when medical fluid is administered to the terminal cancer patient, the second partially open mode is selected in the initial stage. As the pain increases, the administration mode is successively converted from the second partially open mode to the first partially open mode and then to the entirely open mode so that the administration rate of medical fluid increases in stages.

In Figs. 1 through 10, reference numeral 60 denotes a guide which guides the conversion valve 30 contained in the valve chamber 25 so that the conversion valve 30 is correctly operated. Reference numeral 70 denotes a removal prevention means for preventing the conversion valve 30 from being undesirably removed from the chamber 25.

As shown in Figs. 3 through 6, the guide 60 includes a shaft 61 and a guide depression 62 into which the shaft protrusion 61 is inserted. The shaft protrusion 61 is provided on either the bottom of the valve chamber 25 or a lower surface of the conversion valve 30. The guide depression 62 is formed in the other one of the bottom of the valve chamber 25 and the lower surface of the conversion valve 30. In this embodiment, as an example, the case where the shaft protrusion 61 is provided on the bottom of the valve chamber 25 and the guide depression 62 is formed in the lower surface of the conversion valve 30 is illustrated.

As shown in Figs. 4 through 6, the removal prevention means 70 includes a locking protrusion 71 and an annular groove 72 into which the locking protrusion 71 is inserted. The locking protrusion 71 is provided on either a circumferential inner surface of the valve chamber 25 or a circumferential outer surface of the conversion valve 30. The annular groove 72 is formed in the other one of the circumferential inner surface of the valve chamber 25 and the circumferential outer surface of the conversion valve 30. In this embodiment, as an example, the case where the locking protrusion 71 and the annular groove 72 are respectively provided on the circumferential outer surface of the conversion valve 30 and in the circumferential inner surface of the valve chamber 25 in the circumferential direction is illustrated. Moreover, the locking protrusion 71 may comprise a plurality of protrusions which are provided on the circumferential inner surface of the valve chamber 25 or the circumferential outer surface of the conversion valve 30 and are spaced apart from each other at regular intervals in the circumferential direction.

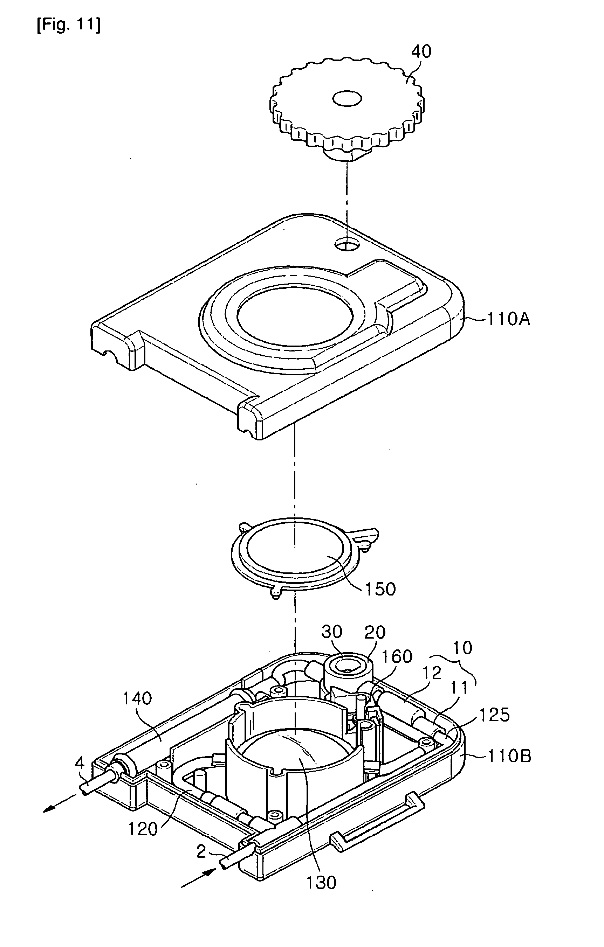

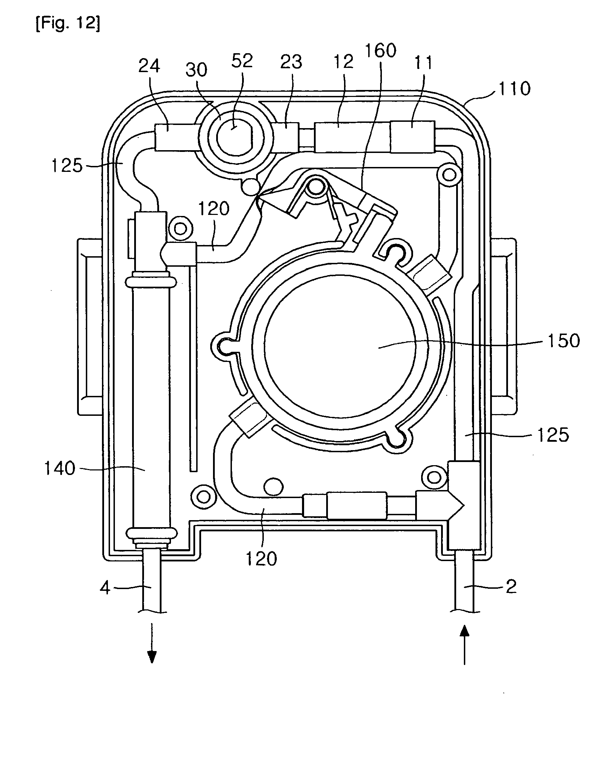

Figs. 11 and 12 are, respectively, an exploded perspective view and a front view showing an example of application of the administration rate control apparatus according to the first embodiment. As shown in Figs. 11 and 12, the administration rate control apparatus according to the first embodiment is provided in casings 110A and 110B of a medical fluid dispenser in such a way that the control handle 40 is exposed to the outside of the casings 110A and 110B.

In detail, the medical fluid dispenser of Figs. 11 and 12 includes first and second branch hoses 120 and 125, a medical fluid pumping bag 130, and a temporary storage and supply means 140. The first and second branch hoses 120 and 125 are bifurcated from an inlet hose 2 and connected to an outlet hose 4. The medical fluid pumping bag 130 is provided on the first branch hose 120 and stores medical fluid that flows along the first branch line therein. When external force is applied to the medical fluid pumping bag 130, it is elastically compressed by the external force to send the stored medical fluid under pressure towards the outlet hose 4. The temporary storage and supply means 140 temporarily stores the medical fluid sent under pressure from the medical fluid pumping bag 130 and continuously discharges the temporarily stored medical fluid through the outlet hose 4 for a predetermined time period. The administration rate control apparatus according to the first embodiment is provided on the second branch hose 125. The inlet tube 11 and the outlet connection port 24 of the administration rate control apparatus are connected to the second branch hose 125.

For reference, medical fluid that is supplied from the inlet hose 2 is drawn into the first and second branch hoses 120 and 125. Medical fluid that is drawn into the second branch hose 125 flows into the outlet hose 4 via the administration rate control apparatus of the first embodiment.

In the drawings, reference numeral 160 denotes a passage opening control unit which opens or closes the passage of the first branch hose 120. Under normal conditions, the first branch hose 120 is closed by the passage opening control unit 160 so that medical fluid that is drawn into the first branch hose 120 is stored in the medical fluid pumping bag 130 rather than flowing into the outlet hose 4.

In this state, when a button designated by reference numeral 150 is pressed, the passage opening control unit 160 opens the passage of the first branch hose 120 and, simultaneously, the medical fluid pumping bag 130 is compressed by the button so that the medical fluid that has been stored in the medical fluid pumping bag 130 is sent under pressure towards the outlet hose 4. Then, an additional amount of medical fluid is momentarily administered to the administration target

The temporary storage and supply means 140 includes a balloon. The medical fluid that is sent under pressure from the first branch hose 120 is drawn into the balloon of the temporary storage and supply means 140 so that the balloon is inflated by the medical fluid drawn therein to temporarily store the medical fluid. Thereafter, the balloon continuously discharges the temporarily stored medical fluid to the outlet hose 4 using contractile force by which the balloon is returned to its original state.

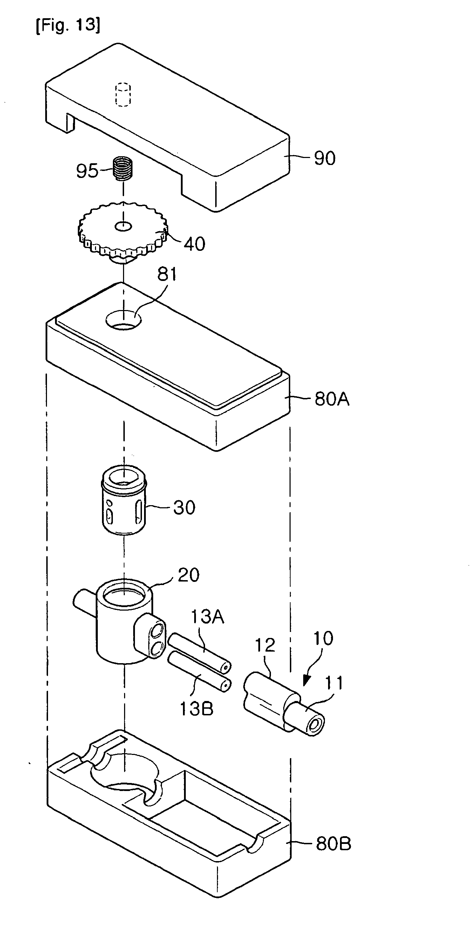

Fig. 13 is an exploded perspective view showing an apparatus for controlling an administration rate of medical fluid, according to a second embodiment of the present invention. As shown in Fig. 13, the general construction and operation of the administration rate control apparatus according to the second embodiment are the same as those of the first embodiment, other than the fact that the second embodiment includes casings 80A and 80b which contain the components except for the control handle 40 and a handle cover 90, whereby the mounting structure of the control handle 40 of the second embodiment differs from that of the first embodiment.

The control handle 40 is connected to the conversion valve 30 through a through hole 81 formed in the upper casing 80A. The handle cover 90 is coupled to the upper casing 80A with the control handle 40 interposed between the handle cover 90 and the upper casing 80A. A spring 95 is interposed between an upper end of the control handle 40 and the handle cover 90. The handle cover 90 is configured in such a way that the circumference of the control handle 40 is exposed out of both sides of the handle cover 90.

Although the preferred embodiments of the present invention have been disclosed for illustrative purposes, those skilled in the art will appreciate that various modifications, additions and substitutions are possible, without departing from the scope of the invention as disclosed in the accompanying claims.

## Claims

1. An apparatus for controlling an administration rate of medical fluid, comprising:
a valve housing (20) having a cylindrical valve chamber (25) therein, with a plurality of medical fluid inlets (21A, 21B) and a medical fluid outlet (22) formed in a circumferential surface of the valve chamber (25);
a conversion valve (30) rotatably contained in the valve chamber (25), the conversion valve (30) having a passage communicating at least one of the medical fluid inlets (21A, 21B) with the medical fluid outlet (22) depending on an angle at which the conversion valve (30) rotates; and
a valve control unit (40) rotating the conversion valve (30),
**characterized in that**
a medical fluid branch unit (10) dividing medical fluid supplied from a medical fluid source into a plurality of streams of medical fluid having different flow rates and supplying the plurality of streams of medical fluid into the respective medical fluid inlets (21A, 21B); and the medical fluid branch unit (10) further comprises:
an inlet tube (11) into which medical fluid is supplied from the medical fluid source; and
a plurality of flow rate control tubes (13A, 13B) diverging from the inlet tube (11), the flow rate control tubes (13A, 13B) being connected to the respective medical fluid inlets (21A, 21B), with passages defined in the respective flow rate control tubes (13A, 13B), the passages having different cross-sectional areas.

2. The apparatus as set forth in claim 1, wherein each of the passages of the flow rate control tubes (13A, 13B) comprises a capillary passage.

3. The apparatus as set forth in claim 1, wherein the medical fluid inlets (21A, 21B) and the medical fluid outlet (22) are disposed opposite to each other,
the passage of the conversion valve (30) comprises a first passage through which the streams of medical fluid supplied from the medical fluid inlets (21A, 21B) flow into the medical fluid outlet (22), and
when the conversion valve (30) is rotated 180º, the first passage is converted to a second passage which prevents the streams of medical fluid supplied from the medical fluid inlets (21A, 21B) from flowing into the medical fluid outlet (22).

4. The apparatus as set forth in claim 3, wherein the passage of the conversion valve (30) further comprises a partially open passage through which medical fluid supplied from some of the medical fluid inlets (21A, 21B) flows into the medical fluid outlet (22).

5. The apparatus as set forth in claim 1, wherein the medical fluid inlets (21A, 21B) comprise first and second inlets, and
the passage of the conversion valve (30) comprises a third passage through which medical fluid supplied from the first inlet (21A) flows into the medical fluid outlet (22), and a fourth passage which prevents medical fluid supplied from the second inlet (21B) from flowing into the medical fluid outlet (22) while the medical fluid flows from the first inlet (21A) into the medical fluid outlet (22) through the third passage.

6. The apparatus as set forth in claim 5, wherein the medical fluid inlets (21A, 21B) and the medical fluid outlet (22) are disposed opposite to each other, and
when the conversion valve (30) is rotated 180º, the third passage and the fourth passage are respectively converted to a fifth passage preventing medical fluid supplied from the first inlet (21A) from flowing into the medical fluid outlet (22), and a sixth passage through which medical fluid supplied from the second inlet (21B) flows into the medical fluid outlet (22).

## Patentansprüche

1. Vorrichtung zum Steuern einer Verabreichungsrate von Arzneifluid, welche umfasst:
ein Ventilgehäuse (20), das eine darin befindliche, zylindrische Ventilkammer (25) mit einer Mehrzahl von Arzneifluideinlässen (21A, 21B) und einem Arzneifluidauslass (22) aufweist, die in einer Umfangsfläche der Ventilkammer (25) ausgebildet sind;
ein Konversionsventil (30), das drehbar in der Ventilkammer (25) enthalten ist, wobei das Konversionsventil (30) einen Durchgang aufweist, der in Abhängigkeit von einer Winkelstellung, in die das Konversionsventil (30) gedreht ist, zumindest einen der Arzneifluideinlässe (21A, 21B) mit dem Arzneifluidauslass (22) in Verbindung setzt; und
eine Ventilsteuereinheit (40), welche das Konversionsventil (30) dreht,
**dadurch gekennzeichnet, dass**
eine Arzneifluid-Verzweigungseinheit (10) von einer Arzneifluidquelle bereitgestelltes Arzneifluid in eine Mehrzahl von Arzneifluidströmen mit unterschiedlichen Durchflussraten aufteilt und die Mehrzahl von Arzneifluidströmen in die entsprechenden Arzneifluideinlässe (21A, 21B) einleitet; und dass die Arzneifluid-Verzweigungseinheit (10) weiterhin umfasst:
ein Einlassrohr (11), in welches Arzneifluid von einer Arzneifluidquelle kommend eingeleitet wird; und
eine Mehrzahl von Durchflussraten-Steuerrohren (13A, 13B), die von dem Einlassrohr (11) abzweigen, wobei die Durchflussraten-Steuerrohre (13A, 13B) mit den jeweiligen Arzneifluideinlässen (21A, 21B) verbunden sind, wobei in den jeweiligen Durchflussraten-Steuerrohren (13A, 13B) Durchgänge definiert sind, wobei die Durchgänge unterschiedliche Querschnittsflächen aufweisen.

2. Vorrichtung nach Anspruch 1, wobei jeder der Durchgänge der Durchflussraten-Steuerrohre (13A, 13B) einen Kapillardurchgang umfasst.

3. Vorrichtung nach Anspruch 1, wobei die Arzneifluideinlässe (21A, 21B) und der Arzneifluidauslass (22) einander gegenüberliegend angeordnet sind,
der Durchgang des Konversionsventils (30) einen ersten Durchgang umfasst, durch den hindurch die von den Arzneifluideinlässen (21A, 21B) bereitgestellten Arzneifluidströme in den Arzneifluidauslass (22) fließen, und
wenn das Konversionsventil (30) um 180 ° gedreht ist, der erste Durchgang zu einem zweiten Durchgang hin umgelenkt wird, der verhindert, dass die von den Arzneifluideinlässen (21A, 21B) bereitgestellten Arzneifluidströme in den Arzneifluidauslass (22) fließen.

4. Vorrichtung nach Anspruch 3, wobei der Durchgang des Konversionsventils (30) weiterhin einen teilweise offenen Durchgang umfasst, durch den das Arzneifluid, das von einigen der Arzneifluideinlässe (21A, 21B) bereitgestellt wird, in den Arzneifluidauslass (22) fließt.

5. Vorrichtung nach Anspruch 1, wobei die Arzneifluideinlässe (21A, 21B) einen ersten und einen zweiten Einlass umfassen, und
der Durchgang des Konversionsventils (30) einen dritten Durchgang umfasst, durch den von dem ersten Einlass (21A) bereitgestelltes Arzneifluid in den Arzneifluidauslass (22) fließt, und einen vierten Durchgang umfasst, welcher verhindert, dass von dem zweiten Einlass (21B) bereitgestelltes Arzneifluid in den Arzneifluid-Auslass (22) fließt, während das Arzneifluid von dem ersten Einlass (21A) durch den dritten Durchgang in den Arzneifluidauslass (22) fließt.

6. Vorrichtung nach Anspruch 5, wobei die Arzneifluideinlässe (21A, 21B) und der Arzneifluidauslass (22) einander gegenüberliegend angeordnet sind, und
wenn das Konversionsventil (30) um 180 ° gedreht ist, der dritte Durchgang und der vierte Durchgang jeweils zu einem fünften Durchgang hin umgelenkt wird, der verhindert, dass von dem ersten Einlass (21A) bereitgestelltes Arzneifluid in den Arzneifluid-Auslass (22) fließt und zu einem sechsten Durchgang durch den von dem zweiten Einlass (21B) bereitgestellten Arzneifluid in den Arzneifluid-Auslass (22) fließt.

## Revendications

1. Dispositif destiné à commander un taux d'administration de fluide médical comprenant:
une cage de soupape (20) qui présente, à l'intérieur, une chambre de soupape cylindrique (25) avec une pluralité d'entrées de fluide médical (21A, 21B) et une sortie de fluide médical (22) lesquelles sont réalisées dans une surface circonférentielle de la chambre de soupape (25);
une soupape de conversion (30) qui est contenue dans la chambre de soupape (25) de manière à pouvoir tourner, la soupape de conversion (30) présentant un passage qui, en fonction de la position angulaire dans laquelle se trouve la soupape de conversion (30), met au moins une des entrées de fluide médical (21A, 21B) en communication avec la sortie de fluide médical (22); et
une unité de commande de soupape (40) qui fait tourner la soupape de conversion (30),
**caractérisé en ce qu'**
une unité de dérivation de fluide médical (10) répartit du fluide médical provenant d'une source de fluide médical en une pluralité de flux de fluide médical de débits différents et fait entrer la pluralité de flux de fluide médical dans les entrées de fluide médical correspondantes (21A, 21B); et **en ce que** l'unité de dérivation de fluide médical (10) comprend en outre:
un tube d'entrée (11) dans lequel est introduit du fluide médical provenant d'une source de fluide médical; et
une pluralité de tubes de commande de débit (13A, 13B) qui partent du tube d'entrée (11), les tubes de commande de débit (13A, 13B) étant reliés aux entrées de fluide médical (21A, 21B), des passages étant définis dans les tubes de commande de débit correspondants (13A, 13B), les passages présentant des surfaces de section différente.

2. Dispositif selon la revendication 1 dans lequel chacun des passages des tubes de commande de débit (13A, 13B) comprend un passage capillaire.

3. Dispositif selon la revendication 1 dans lequel les entrées de fluide médical (21A, 21B) et la sortie de fluide médical (22) sont disposées de manière opposée,
le passage de la soupape de conversion (30) comprend un premier passage que traversent les flux de fluide médical provenant des entrées de fluide médical (21A, 21B) pour passer dans la sortie de fluide médical (22), et
lorsque la soupape de conversion (30) est tournée de 180 °, le premier passage est déplacé vers un deuxième passage qui empêche les flux de fluide médical provenant des entrées de fluide médical (21A, 21B) de passer dans la sortie de fluide médical (22).

4. Dispositif selon la revendication 3 dans lequel le passage de la soupape de conversion (30) comprend en outre un passage partiellement ouvert que traverse du fluide médical provenant de quelques-unes des entrées de fluide médical (21A, 21B) pour passer dans la sortie de fluide médical (22).

5. Dispositif selon la revendication 1 dans lequel les entrées de fluide médical (21A, 21B) comprennent une première et une deuxième entrée, et
le passage de la soupape de conversion (30) comprend un troisième passage que traverse du fluide médical provenant de la première entrée (21A) pour passer dans la sortie de fluide médical (22), et comprend un quatrième passage qui empêche du fluide médical provenant de la deuxième entrée (21B) de passer dans la sortie de fluide médical (22) alors que le fluide médical de la première entrée (21A) passe, via le troisième passage, dans la sortie de fluide médical (22).

6. Dispositif selon la revendication 5 dans lequel les entrées de fluide médical (21A, 21B) et la sortie de fluide médical (22) sont disposées de manière opposée, et
lorsque la soupape de conversion (30) est tournée de 180 °, le troisième passage et le quatrième passage sont déplacés respectivement vers un cinquième passage qui empêche du fluide médical provenant de la première sortie (21A) de passer dans la sortie de fluide médical (22) et vers un sixième passage qui traversent du fluide médical provenant de la deuxième entrée (21B) de passer dans la sortie de fluide médical (22).
